# EUROPEAN PATENT APPLICATION

(11) **EP 1 111 045 A2**
(43) Date of publication of application: **27.06.2001**
(21) Application number: 00204396.6
(22) Date of filing: 08.12.2000
(51) Int. Cl.: C12N 15/12, C07K 14/47, C07K 16/18, A61K 38/17, A61K 48/00

(54) **Apoptin-associating protein**

(30) Priority: 10.12.1999 EP 99204242; 07.04.2000 EP 00250119
(71) Applicant: Leadd B.V., 2333 AL Leiden (NL)
(72) Inventor: Danen-van Oorschot, Astrid Adriana Anna Maria, 2651 VG Berkel en Rodenrijs (NL); Weiss, Bertram, 14195 Berlin (DE); Noteborn, Mathieu Hubertus Maria, 2352 EH Leiderdorp (NL); Rohn, Jennifer Leigh, 1074 BR Amsterdam (NL); Toschi, Luisella, 10589 Berlin (DE)
(74) Representative: Prins, Adrianus Willem

(57) **Abstract**

The invention relates to the field of apoptosis. The invention provides novel therapeutic possibilities, for example novel combinatorial therapies or novel therapeutic compounds that can work alone, sequentially to, or jointly with Apoptin, especially in those cases wherein p53 is (partly) non-functional.

## Description

### The invention relates to the field of apoptosis.

Apoptosis is an active and programmed physiological process for eliminating superfluous, altered or malignant cells (Earnshaw, 1995, Duke et al., 1996). Apoptosis is characterized by shrinkage of cells, segmentation of the nucleus, condensation and cleavage of DNA into domain-sized fragments, in most cells followed by internucleosomal degradation. The apoptotic cells fragment into membrane-enclosed apoptotic bodies. Finally, neighbouring cells and/or macrophages will rapidly phagocytose these dying cells (Wyllie et al., 1980, White, 1996). Cells grown under tissue-culture conditions and cells from tissue material can be analysed for being apoptotic with agents staining DNA, as e.g. DAPI, which stains normal DNA strongly and regularly, whereas apoptotic DNA is stained weakly and/or irregularly (Noteborn et al., 1994, Telford et al., 1992).

The apoptotic process can be initiated by a variety of regulatory stimuli (Wyllie, 1995, White 1996, Levine, 1997). Changes in the cell survival rate play an important role in human pathogenesis of diseases, e.g. in cancer development and auto-immune diseases, where enhanced proliferation or decreased cell death (Kerr et al., 1994, Paulovich, 1997) is observed. A variety of chemotherapeutic compounds and radiation have been demonstrated to induce apoptosis in tumor cells, in many instances via wild-type p53 protein (Thompson, 1995, Bellamy et al., 1995, Steller, 1995, McDonell et al., 1995).

Many tumors, however, acquire a mutation in p53 during their development, often correlating with poor response to cancer therapy. Certain transforming genes of tumorigenic DNA viruses can inactivate p53 by directly binding to it (Teodoro, 1997). An example of such an agent is the large T antigen of the tumor DNA virus SV40. For several (leukemic) tumors, a high expression level of the proto-oncogene Bcl-2 or Bcr-abl is associated with a strong resistance to various apoptosis-inducing chemotherapeutic agents (Hockenberry 1994, Sachs and Lotem, 1997).

For such tumors lacking functional p53 (representing more than half of the tumors) alternative anti-tumor therapies are under development based on induction of apoptosis independent of p53 (Thompson 1995, Paulovich et al., 1997). One has to search for the factors involved in induction of apoptosis, which do not need p53 and/or can not be blocked by anti-apoptotic activities, such as Bcl-2 or Bcr-abl-like ones. These factors might be part of a distinct apoptosis pathway or might be (far) downstream of the apoptosis inhibiting compounds.

Apoptin is a small protein derived from chicken anemia virus (CAV; Noteborn and De Boer, 1995, Noteborn et al., 1991, Noteborn et al., 1994; 1998a), which can induce apoptosis in human malignant and transformed cell lines, but not in untransformed human cell cultures. *In vitro,* Apoptin fails to induce programmed cell death in normal lymphoid, dermal, epidermal, endothelial and smooth-muscle cells. However, when normal cells are transformed they become susceptible to apoptosis by Apoptin. Long-term expression of Apoptin in normal human fibroblasts revealed that Apoptin has no toxic or transforming activity in these cells (Danen-van Oorschot, 1997 and Noteborn, 1996).

In normal cells, Apoptin was found predominantly in the cytoplasm, whereas in transformed or malignant cells i.e. characterized by hyperplasia, metaplasia ordysplasia, it was located in the nucleus, suggesting that the localization of Apoptin is related to its activity (Danen-van Oorschot et al. 1997).

Apoptin-induced apoptosis occurs in the absence of functional p53 (Zhuang et al., 1995a), and cannot be blocked by Bcl-2, Bcr-abl (Zhuang et al., 1995), or the Bcl-2-associating protein BAG-1 (Danen-Van Oorschot, 1997a, Noteborn, 1996).

Therefore, Apoptin is a therapeutic compound for the selective destruction of tumor cells, or other hyperplasia, metaplasia or dysplasia, especially for those tumor cells which have become resistant to (chemo)-therapeutic induction of apoptosis, due to the lack of functional p53 and (over)-expression of Bcl-2 and other apoptosis-inhibiting agents (Noteborn and Pietersen, 1998). It appears, that even pre-malignant, minimally transformed cells, are sensitive to the death-inducing effect of Apoptin. In addition, Noteborn and Zhang (1998) have shown that Apoptin-induced apoptosis can be used as diagnosis of cancer-prone cells and treatment of cancer-prone cells.

The fact that Apoptin does not induce apoptosis in normal human cells, at least not *in vitro,* shows that a toxic effect of Apoptin treatment *in vivo* will be very low. Noteborn and Pietersen (1998) and Pietersen et al. (1999) have provided evidence that adenovirus expressed Apoptin does not have an acute toxic effect *in vivo.* In addition, in nude mice it was shown that Apoptin has a strong anti-tumor activity.

However, to further enlarge the array of therapeutic anti-cancer or anti-auto-immune-disease compounds available in the art, additional therapeutic compounds are desired that are designed to work alone, sequentially to, or jointly with Apoptin, especially in those cases wherein p53 is (partly) non-functional.

The invention provides novel therapeutic possibilities, for example novel combinatorial therapies or novel therapeutic compounds that can work alone, sequentially to, or jointly with Apoptin, especially in those cases wherein p53 is (partly) non-functional.

In a first embodiment, the invention provides an isolated or recombinant nucleic acid or functional equivalent or fragment thereof encoding an Apoptin-associating proteinaceous substance capable of nuclear localisation, or nuclear co-localisation with Apoptin, and is capable of providing apoptosis, alone or in combination with other apoptosis inducing substances, such as Apoptin, particularly in transformed cells or tumorous cells. In a preferred embodiment it co-localizes with chromatin/DNA structures in the nucleus of the cell, in a initial apoptotic phase leading up to segmentation of the nucleus, condensation and cleavage of DNA into fragments, in most of said cells followed by internucleosomal degradation. In another preferred embodiment of the invention, said substance allows co-localisation which takes place in a somewhat organised pattern whereby circular structures are formed in said nucleus, particularly in an area of the nucleus containing heterochromatin. However, during the above described apoptotic segmentation of the nucleus by condensation and cleavage of DNA into fragments, the euchromatin is of course also effected.

Proteinaceous substance herein is defined as a substance comprising a peptide, polypeptide or protein, optionally having been modified by for example glycosylation, myristilation, phosphorylation, the addition of lipids, by homologous or heterologous di-or multimerisation, or any other (posttranslational) modifications known in the art.

Apoptin-associating herein is defined as belonging to the cascade of substances specifically involved in the cascade of events found in the apoptosis pathway as inducable by Apoptin, preferably those substances that are specifically involved in the p53-independent apoptosis pathway.

In a preferred embodiment, the invention provides an isolated or recombinant nucleic acid or functional equivalent or fragment thereof encoding an Apoptin-associating proteinaceous substance capable of providing apoptosis derived from a cDNA library, preferably a vertebrate cDNA library, such as derivable from poultry, but more preferably a mammalian cDNA library, preferably wherein said cDNA library comprises human cDNA. An Apoptin-associating proteinaceous substance obtained by determining a vertebrate analogue (preferably human) of an Apoptin-associating proteinaceous substance derived from an invertebrate cDNA library is also included.

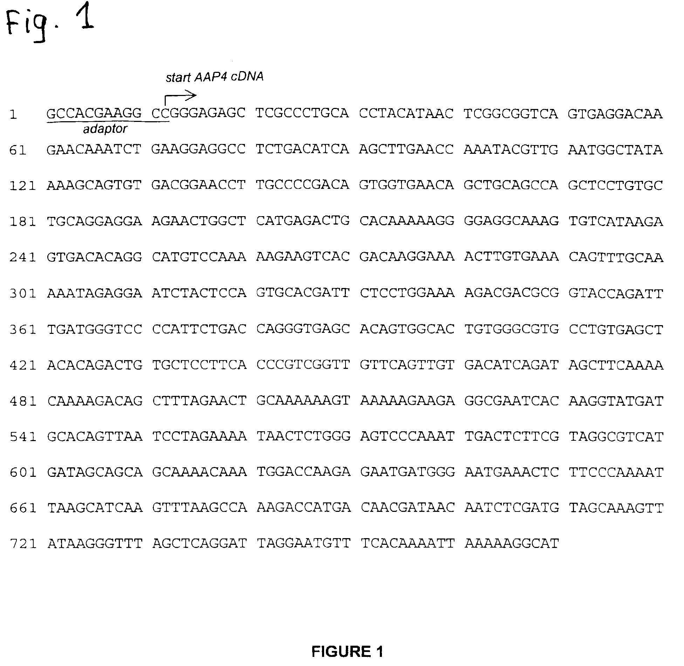

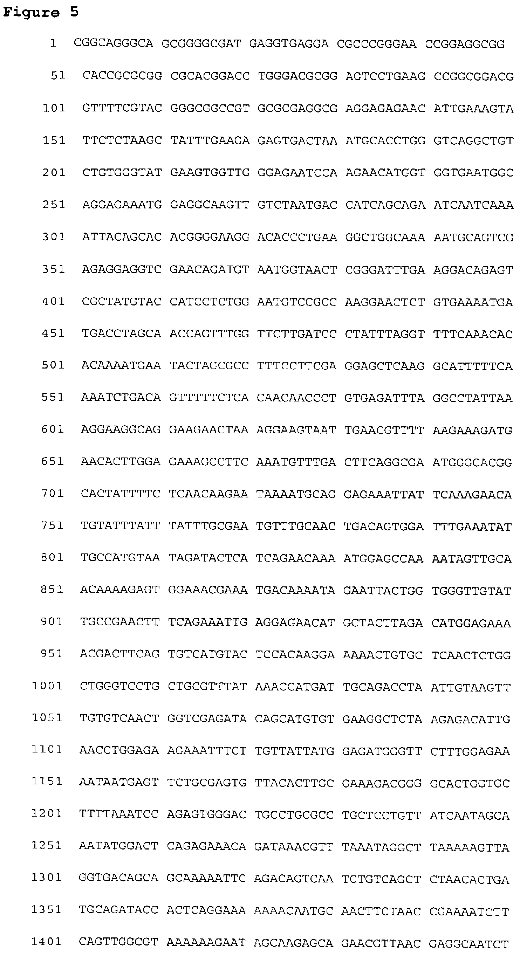

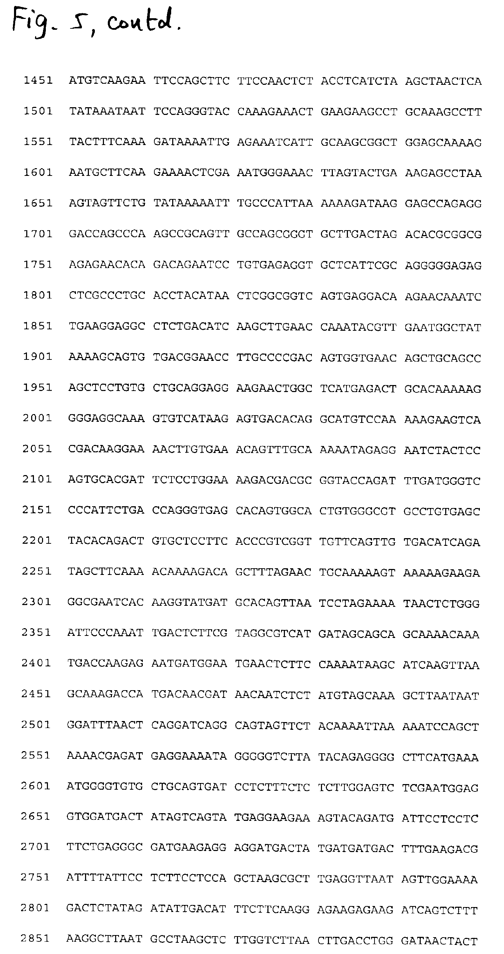

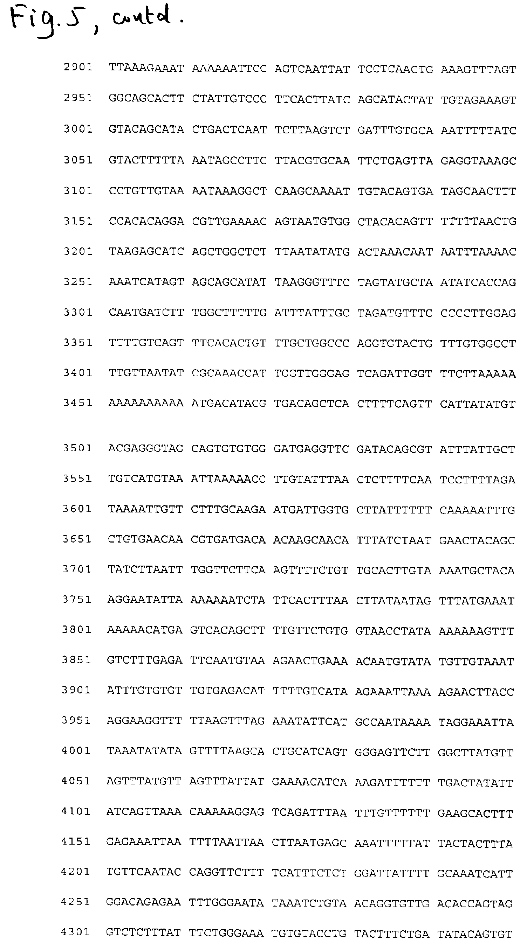

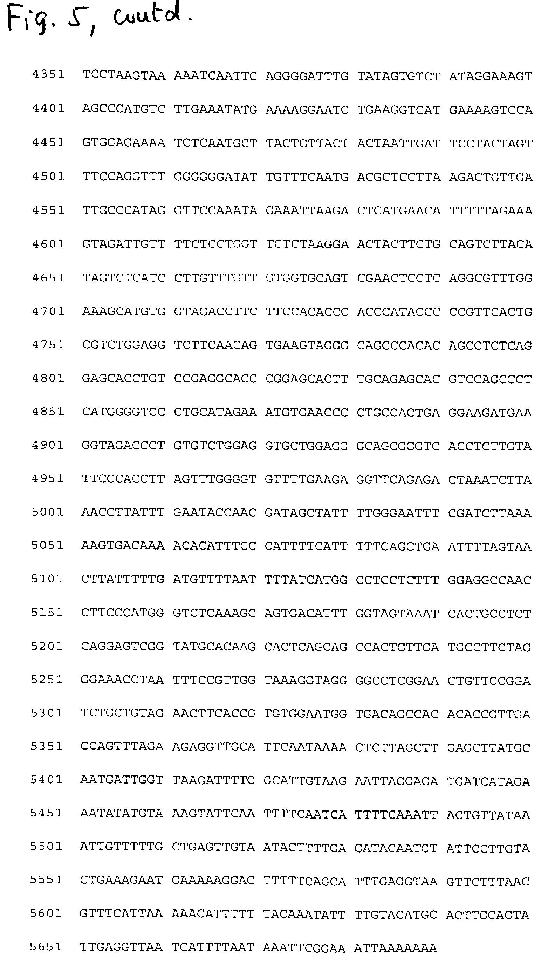

In another embodiment, the invention provides an isolated or recombinant nucleic acid or functional equivalent or fragment thereof encoding an Apoptin-associating proteinaceous substance capable of providing apoptosis capable of hybridising to a nucleic acid molecule encoding an Apoptin-associating proteinaceous substance capable of providing apoptosis as shown in figure 1 or 5, in particular encoding a novel protein capable of providing apoptosis or functional equivalent or functional fragment thereof called Apoptin-associating protein 4, abbreviated herein also as AAP-4. Figure 1 shows an approximately 750 bp fragment of the complete AAP-4 fragment as depicted in Figure 5. Both nucleotide sequence encode an protein with at least the capability of binding to Apoptin and providing apoptosis. Of course, an isolated or recombinant nucleic acid or functional equivalent or fragment thereof encoding an additional Apoptin-associating proteinaceous substance capable of associating with the partial or complete AAP-4 protein are herewith also provided, means and methods to arrive at such an additional protein located in the Apoptin cascade follow those of the detailed description given herein. Knowledge derived from studying the partial or full-length AAP-4 is exploited to determine a functional pathway in which partial or full-length AAP-4 is involved, thus allowing the design of a therapeutic means of intervention in such a pathway.

In particular, the invention provides an isolated or recombinant nucleic acid or functional equivalent or fragment thereof encoding an Apoptin-associating proteinaceous substance capable of providing apoptosis being at least 60% homologous, preferably at least 70%, more preferably at least 80%, even more preferably 90% and most preferably at least 95% homologous to a nucleic acid molecule, or to a functional equivalent or functional fragment thereof, encoding an Apoptin-associating proteinaceous substance as shown in figure 1 or 5.

Furthermore, the invention provides a vector comprising a nucleic acid according to the invention. Examples of such a vector are given in the detailed description given herein; such as vector pMT2SM-AAP-4, pMT2SM vector expressing Myc-tagged AAP-4 cDNA, a plasmid expressing an Apoptin-associating protein fragment, and so on. These and other vectors are for example useful in finding additional Apoptin-associating proteinaceous substances from the cascade, as defined above.

In yet another embodiment, the invention provides a vector comprising a nucleic acid according to the invention, said vector comprising a gene-delivery vehicle, making the invention very useful in gene therapy. By equiping a gene delivery vehicle with a nucleic acid according to the invention, and by targeting said vehicle to a cell or cells that have been over-proliferating and/or have shown decreased death rates, said gene delivery vehicle provides said cell or cells with the necessary means for apoptosis, providing far reaching therapeutic possibilities.

Furthermore, the invention provides a host cell comprising a nucleic acid or a vector according to the invention. Examples comprise transformed or transfected bacterial or yeast cells as described in the detailed description herein. Preferred is a host cell according to the invention which is a transformed eukaryotic cell such as a yeast cell or a vertebrate cell, such as mammalian or Cos cells transformed or transfected with a nucleic acid or vector according to the invention. Said cells are in general capable to express or produce a proteinaceous substance capable of providing apoptosis with the ability to associate with Apoptin.

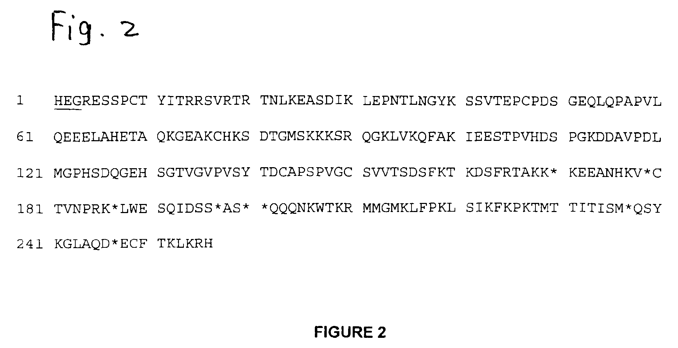

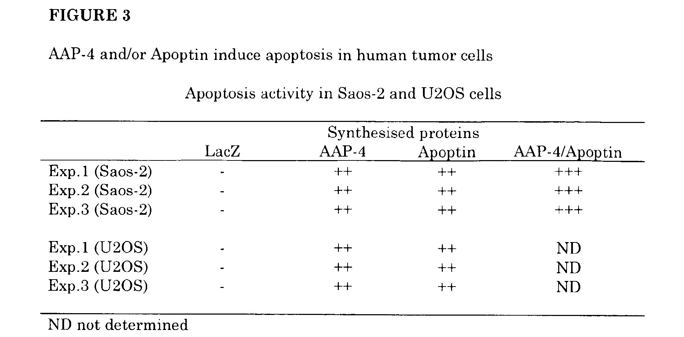

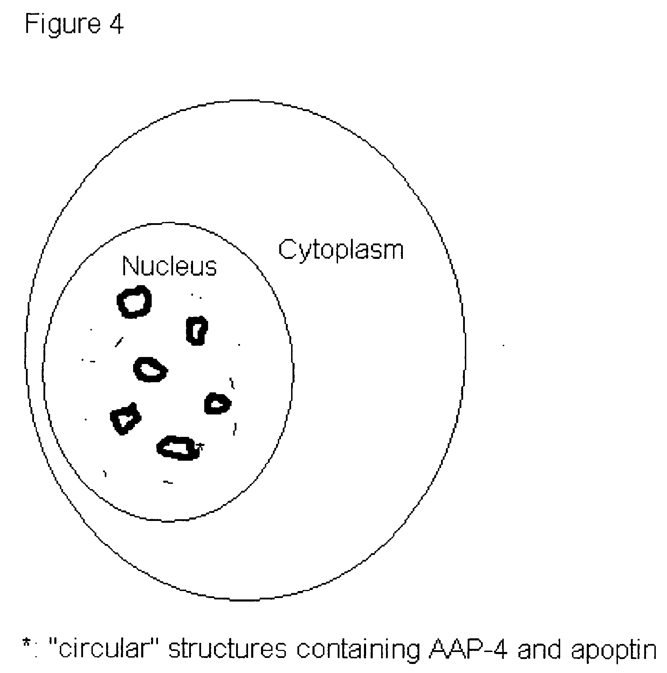

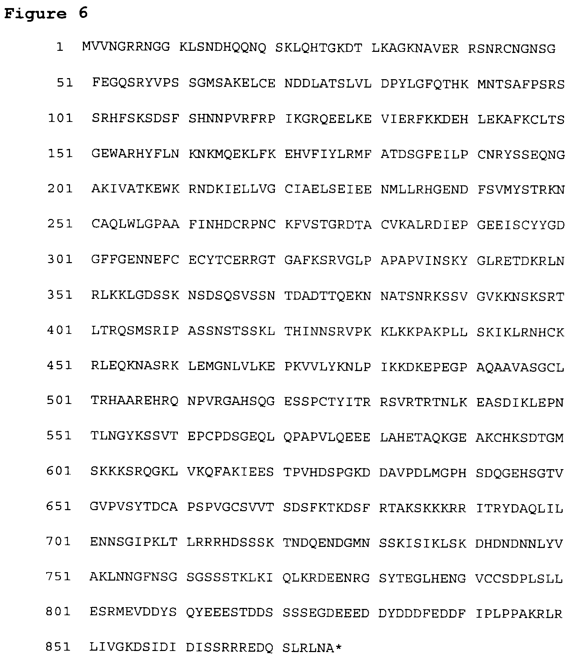

The invention furthermore provides an isolated or recombinant Apoptin-associating proteinaceous substance capable of providing apoptosis. As for example shown herein in figure 3, expression of such Apoptin-associating proteinaceous substance in cells, such as tumour cells, or other over-proliferating cells, induces the apoptotic process. It can do so alone, or in the presence of other apoptosis inducing substances such as Apoptin, and especially so independent of p53, showing that also in those cases where (functional) p53 is absent apoptosis can be induced by a substance according to the invention. In particular, the invention provides a proteinaceous substance according to the invention encoded by a nucleic acid according to the invention, for example comprising at least a part of an amino acid sequence as shown in figure 2 or 6 or a functional equivalent or functional fragment thereof capable of providing apoptosis alone or in combination, preferably co-localising, with other apoptosis inducing substances such as Apoptin (see for example figure 4, where circular structures are shown where Apoptin and a substance as provided were found to co-localise in transformed or tumorous cells).

The invention also provides an isolated or synthetic antibody specifically recognising a proteinaceous substance or functional equivalent or functional fragment thereof according to the invention. Such an antibody is for example obtainable by immunising an experimental animal with a Apoptin-associating proteinaceous substance or an immunogenic fragment or equivalent thereof and harvesting polyclonal antibodies from said immunised animal (as shown herein in the detailed description), or obtainable by other methods known in the art such as by producing monoclonal antibodies, or (single chain) antibodies or binding proteins expressed from recombinant nucleic acid derived from a nucleic acid library, for example obtainable via phage display techniques.

With such an antibody, the invention also provides a proteinaceous substance specifically recognisable by such an antibody according to the invention, for example obtainable via immunoprecipitation, Western Blotting, or other immunological techniques known in the art.

Furthermore, the invention provides use of a nucleic acid, vector, host cell, or proteinaceous substance according to the invention for the induction of apoptosis, as for example shown in figure 3. In particular, such use is provided wherein said apoptosis is p53-independent. In particular, such use is also provided further comprising use of a nucleic acid encoding Apoptin or a functional equivalent or fragment thereof or use of Apoptin or a functional equivalent or fragment thereof. As can be seen from figure 3, combining these Apoptin-inducing substances increases the percentage apoptosis of treated tumour cells.

Such use as provided by the invention is particularly useful from a therapeutic viewpoint. The invention provides herewith a pharmaceutical composition comprising a nucleic acid, vector, host cell, or proteinaceous substance according to the invention. In addition, such a pharmaceutical composition according to the invention is provided further comprising a nucleic acid encoding Apoptin or a functional equivalent or fragment thereof or Apoptin or a functional equivalent or fragment thereof.

Such a pharmaceutical composition is in particular provided for the induction of apoptosis, for example wherein said apoptosis is p53-independent, for the treatment of a disease where enhanced cell proliferation or decreased cell death is observed, as is in general the case when said disease comprises cancer or auto-immune disease. Herewith the invention provides a method for treating an individual carrying a disease where enhanced cell proliferation or decreased cell death is observed comprising treating said individual with a pharmaceutical composition according to the invention. In particular these compositions comprise a factor of an apoptosis pathway, which is specific for transformed cells and cancer-prone cells. Therefore, these compositions are essential for new treatments, but also for diagnosis of diseases related with aberrance's in the apoptotic process, such as cancer and auto-immune diseases. Furthermore, the invention provides for diagnosis of cancer-prone cells in particular by detecting those cells that under influence of a substance (e.g. by transfection) as provided by the invention show condensing of chromatin/DNA or circular structures as described in figure 4.

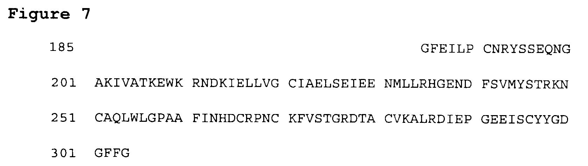

The invention also provides an isolated or recombinant nucleic acid encoding a proteinaceous substance comprising the amino acid sequence as shown in figure 7.

In a further embodiment the invention provides an assay to identify a putative effector of the activity of the proteinaceous substance encoded by a nucleic acid as shown in figure 5 comprising bringing in contact a proteinaceous substance comprising amino acid 852-900 of the amino acid sequence shown in figure 6 with said effector and determining the binding of said effector.

The invention will be explained in more detail in the following detailed description which is not limiting the invention.

### Detailed description

We have used the yeast-2 hybrid system (Durfee et al., 1993) to identify Apoptin-associating cellular compounds, which are essential in the induction of apoptosis. The used system is an *in vivo* strategy to identify human proteins capable of physically associating with Apoptin. It has been used to screen cDNA libraries for clones encoding proteins capable of binding to a protein of interest (Fields and Song, 1989, Yang et al., 1992). The invention provides for example a novel Apoptin-associating protein, one of which is named Apoptin-associating protein 4 abbreviated as AAP-4. The invention also provides a method for inducing apoptosis through interference with the function of this newly discovered AAP-4 protein or functional equivalents or fragments thereof and/or the induction of apoptosis by means of (over)expression of AAP-4 or related gene or functional equivalents or fragments thereof.

The invention also provides an anti-tumor therapy based on the interference with the function of AAP-4-like proteins and/or its (over)expression. An aberrant high level of AAP-4-like proteins will result in the induction of the opposite process of cell transformation, namely apoptosis. The invention furthermore provides the mediator of Apoptin-induced apoptosis, which is tumor-specific. The invention provides a therapy for cancer, auto-immune diseases or related diseases which is based on AAP-4-like proteins alone or in combination with Apoptin and/or Apoptin-like compounds.

### Construction of pGBT9-VP3

For the construction of the bait plasmid, which enables the identification of Apoptin-associating proteins by means of a yeast-two-hybrid system, plasmid pET-16b-VP3 (Noteborn, unpublished results) was treated with NdeI and BamHI. The 0.4 kb NdeI-BamHI DNA fragment was isolated from low-melting-point agarose.

Plasmid pGBT9 (Clontech Laboratories, Inc, Palo Alto, USA) was treated with the restriction enzymes EcoRI and BamHI. The about 5.4-kb DNA fragment was isolated and ligated to an EcoRI-NdeI linker and the 0.4-kb DNA fragment containing the Apoptin-encoding sequences starting from its own ATG-initiation codon. The final construct containing a fusion gene of the GAL4-binding domain sequence and Apoptin under the regulation of the yeast promoter ADH was called pGBT-VP3 and was proven to be correct by restriction-enzyme analysis and DNA-sequencing according to the Sanger method (1977).

All cloning steps were essentially carried out as described by Maniatis et al. (1992). The plasmid pGBT-VP3 was purified by centrifugation in a CsCl gradient and column chromatography in Sephacryl S500 (Pharmacia).

### GAL4-activation domain-tagged cDNA library

The expression vector pACT, containing the cDNAs from Epstein-Barr-virus-transformed human B cells fused to the GAL4 transcriptional activation domain, was used for detecting Apoptin-associating proteins. The pACT c-DNA library is derived from the lambda-ACT cDNA library, as described by Durfee et al. 1993.

### Bacterial and Yeast strains

The E.coli strain JM109 was the transformation recipient for the plasmid pGBT9 and pGBT-VP3. The bacterial strain electromax/DH10B was used for the transformation needed for the recovery of the Apoptin-associating pACT-cDNAs, and was obtained from GIBCO-BRL, USA.

The yeast strain Y190 was used for screening the cDNA library, and all other transformations, which are part of the used yeast-two-hybrid system.

### Media

For drug selections Luria Broth (LB) plates for E.coli were supplemented with ampicillin (50 microgram per ml). Yeast YPD and SC media were prepared as described by Rose et al. (1990).

### Transformation of competent yeast strain Y190 with plasmids pGBT-VP3 and pACT-cDNA and screening for beta-galactosidase activity.

The yeast strain Y190 was made competent and transformed according to the methods described by Klebe et al. (1983). The yeast cells were first transformed with pGBT-VP3 and subsequently transformed with pACT-cDNA, and these transformed yeast cells were grown on histidine-minus plates, also lacking leucine and tryptophan.

Hybond-N filters were placed on yeast colonies, which were histidine-positive and allowed to wet completely. The filters were lifted and submerged in liquid nitrogen to permeabilize the yeast cells. The filters were thawed and layed with the colony side up on Whattman 3MM paper in a petridish with Z-buffer (Per liter: 16.1 gr Na₂HPO₄.7H₂O, 5.5 gr NaH₂PO₄.H₂O, 0.75 gr KCl and 0,246 gr MgSO₄.7H₂O, pH 7.0) containing 0.27% beta-mercapto-ethanol and 1 mg/ml X-gal. The filters were incubated for at least 15 minutes or during night.

### Recovery of plasmids from yeast

Total DNA from yeast cells, which were histidine- and beta-galactosidase-positive, was prepared by using the glusulase-alkaline lysis method as described by Hoffman and Winston (1987) and used to transform Electromax/DH10B bacteria via electroporation using a Bio-Rad GenePulser according the manufacturer's specifications.
Transformants were plated on LB media containing the antibiotic agent ampicillin.

### Isolation of Apoptin-associating pACT clones

By means of colony-filter assay the colonies were lysed and hybridized to a radioactive-labeled 17-mer oligomer, which is specific for pACT (see also section Sequence analysis). Plasmid DNA was isolated from the pACT-clones, and by means of XhoI digestion analysed for the presence of a cDNA insert.

### Sequence analysis

The subclone containing the sequence encoding Apoptin-associating protein was partially sequenced using dideoxy NTPs according to the Sanger-method, which was performed by Eurogentec, Seraing, Belgium). The used sequencing primer was a pACT-specific 17-mer comprising of the DNA-sequence 5'-TACCACTACAATGGATG-3'.

The sequences of the Apoptin-associating cDNAs were compared with known gene sequences from the EMBL/Genbank.

### Generation and testing of antibodies.

In order to generate polyclonal antisera against the AAP-4 protein, we designed three peptides. These peptides were:
1) EESTPVHDSPGKDDA
2) DSFKTKDSFRTAKSK
3) IDIDISSRRREDQSL
These peptides were synthesized at Eurogentec (Belgium) with the standard addition of a C-terminal cysteine residue and all subsequent antibody syntheses was also performed there. These peptides were coupled to Keyhole Limpet Hemocyanin (KLH) and injected as a cocktail into two separate specific pathogen free rabbits with an immunization schedule of one injection and three subsequent boosts. Blood samples were taken before and after immunization. The sera were tested in-house for specific reactivity to the peptide cocktail by ELISA. The titers from each rabbit were high (>200,000). Furthermore, for certain subsequent purposes, the AAP-4 antibody was immune-purified using peptide cocktail coupled to immobilized diaminodipropylamine agarose columns (Pierce) according to the manufacturer's protocol.

The best AAP-4 antibody preparation of the two generated was selected for further use. We tested the efficacy of this antibody by transfecting 6 cm plates of sub-confluent primate COS-7 and human U20S cells using the calcium phosphate co-precipitation method with 5 ug of the AAP-4-myc construct, and as a control, untransfected cells. Two days post-transfection, cells were washed briefly in PBS, lysed in RIPA buffer (10 mM Tris 7.5, 150 mM NaCl, 0.1% SDS, 1.0% NP-49 and 1.0 % sodium deoxycholate), clarified by centrifugation, and the supernatant fractionated on SDS-denaturing polyacrylamide gel electrophoresis. Proteins were Western-transferred to PVDF membranes (Immobilon, Millipore) using standard methodology. Membranes were blocked in 5% non-fat dry milk in tris-buffered saline containing 0.1% Tween-20, then incubated in the unpurified AAP-4 antisera at a concentration of 1:5000. After a brief wash, membranes were further incubated in HRP-conjugated goat-anti-rabbit Ig at a concentration of 1:2000. After a thorough series of wash steps, proteins were detected using enhanced chemiluminescence (Amersham) according to the manufacturer's protocol, and exposed to x-ray film and developed using standard automated machinery.

In addition, we tested the purified AAP-4 antibody using immunoprecipitation in a manner the same as above, except that after centrifugation, the supernatant was added to 10 ul of AAP-4 antibody precoupled to protein-A-sepharose beads, incubated for 1 hour with tumbling, then washed before fractionation on SDS-PAGE gels and Western analysis. Detection in this case was performed with the anti-myc tag monoclonal antibody 9E10 (Evan et al. 1985).

Finally, the purified antibody was tested for utility in immunofluorescence by including glass coverslips in the above transfections. Coverslips were fixed with 4% paraformaldehyde, blocked with normal goat serum, incubated in AAP-4 antibody diluted 1:5, washed, incubated in FITC-conjugated goat-anti-rabbit Ig, mounted and visualized under fluorescence microscopy.

### Northern blot analysis

To examine whether AAP-4 was differentially expressed in tumor versus normal tissue, we tested a commercially-available Northern blot (Invitrogen, cat. No. #D310001) that contained tumor and normal tissue derived from the same patient, from a variety of tissue types. The DNA probe was derived from an internal HindIII fragment of AAP-4 and was labelled with ³²P-dATP using the MegaPrime kit of Amersham. All prehybridization, hybridization and washing steps were done according to the Northern blot manufacturer's recommendation. Blots were subjected to autoradiography and developed using standard automated methods.

### Cloning of full-length AAP-4

A human brain cDNA library was obtained from Clontech (Marathon-Ready^{TM} cDNA). The cDNA for AAP-4 was generated in a RACE-PCR (Rapid Amplification of cDNA Ends) according to the manufacturer's instructions included in the Marathon-Ready^{TM} cDNA kit.

For the RACE-PCR the following "touch-down" program was used in a Perkin-Elmer 9600 thermocycler: 1 cycle 94°C 30 sec; 5 cycles of 94°C 5 sec, 72°C 3 min; 5 cycles of 94°C 5 sec, 70°C 3 min; 25 cycles of 94°C 5 sec, 68°C 3 min. The sequences of the AAP-4 primers used in the RACE reaction were: 
AAP-4#3F 5' GTC AGC TCT AAC ACT GAT GCA GAT ACC AC 3'
AAP-4#3R 5' GTG GTA TCT GCA TCA GTG TTA GAG CTG AC 3'
 The RACE products were cloned in the pCR®4-TOPO vector according to the instructions of the TOPO-TA cloning kit from Invitrogen. The sequences of the cloned PCR products were amplified with the Applied Biosystem (ABI) Prism®BigDye^{TM} Terminator sequencing kit and analysed in the ABI 310 capillary sequencer. The complete open reading frame (ORF) of AAP-4 was subsequently amplified in the following PCR reaction: 1 cycle 94°C 30 sec; 30 cycles 94°C 5 sec, 68°C 3 min. The gene-specific primers had the following sequences: 
AAP-4#8F 5' GAG AGT GAC TAA ATG CAC CTG GGT CAG G 3'
AAP-4#9R 5' GTT ATC CCA GGT CAA GTT AAG ACC 3'
 The full-length generated product cloned into the pCR®4-TOPO vector was subjected to a final sequence analysis as previously described.

### Results and discussion

Apoptin induces apoptosis specifically in transformed cells, such as cell lines derived from human tumors. To identify the essential compounds in this cell-transformation-specific and/or tumor-specific apoptosis pathway, a yeast genetic screen was carried out.
We have used a human cDNA library, which is based on the plasmid vector pACT containing the complete cDNA copies made from Epstein-Barr virus-transformed human B cells (Durfee et al., 1993).

### Construction of a bait plasmid expressing a fusion gene product of GAL4-DNA-binding domain and Apoptin

To examine the existence of Apoptin-associating proteins in the human transformed/tumorigenic cDNA library, a so-called bait plasmid had to be constructed. To that end, the complete Apoptin-encoding region, flanked by about 40 basepairs downstream from the Apoptin gene, was cloned in the multiple cloning site of plasmid pGBT9.

The final construct, called pGBT-VP3, was analysed by restriction-enzyme analysis and sequencing of the fusion area between Apoptin and the GAL4-DNA-binding domain.

### A gene(fragment) encoding an Apoptin-associating protein is determined by transactivation of a GAL4-responsive promoter in yeast.

The Apoptin gene was fused to the GAL4-DNA-binding domain of plasmid pGBT-VP3, whereas all cDNAs derived from the transformed human B cells are fused to the GAL4-activation domain of plasmid pACT. If one of the proteinaceous substances encoded by said cDNAs binds to Apoptin, the GAL4-DNA-binding domain would be in the vicinity of the GAL4-activation domain resulting in the activation of the GAL4-responsive promoter, which regulates the reporter genes HIS3 and LacZ.

The yeast clones containing plasmid expressing Apoptin and a plasmid expressing an Apoptin-associating protein fragment can grow on a histidine-minus medium and will stain blue in a beta-galactosidase assay. Subsequently, the plasmid with the cDNA insert encoding the Apoptin-associating protein can be isolated and characterized.

Before we did so, however, we determined that transformation of yeast cells with pGBT-VP3 plasmid alone, or in combination with an empty pACT vector, did not result in the activation of the GAL4-responsive promoter.

### Identification of Apoptin-associating protein encoded by cDNA derived from a human transformed B cell line.

We have found one yeast colony that upon transformation with pGBT-VP3 and pACT-cDNA was able to grow on a histidine-minus medium (also lacking leucine and tryptophan) and stained blue in a beta-galactosidase assay. These results indicate that the observed yeast colony contained besides the bait plasmid pGBT-VP3 also a pACT plasmid encoding a potential Apoptin-associating protein.

Plasmid DNA was isolated from the positive yeast colonyand transformed in bacteria. By means of a filter-hybridization assay using a pACT-specific labeled DNA-probe, the clone containing pACT plasmid could be determined. Subsequently, pACT DNA was isolated and digested with restriction enzyme XhoI, which resulted in the presence of a 2.1-kbp cDNA insert. Finally, the insert of pACT plasmid containing the cDNA insert was fully sequenced by using the Sanger method (Sanger et al., 1977).

### Description of Apoptin-associating proteins

The yeast genetic screen for Apoptin-associating proteins resulted in the detection of a cDNA clone comprising a single type of protein, namely a novel protein called Apoptin-associating protein 4, abbreviated as AAP-4.

The determined DNA sequence part of the AAP-4 cDNA clone is shown in Figure 1. The amino acid sequence, derived from the detected DNA sequence of clone AAP-4 is given in Figure 2.

### Construction of an expression construct for the identification of AAP-4 protein in mammalian cells.

To study whether the cloned cDNA AAP-4 indeed encodes (Apoptin-associating) a protein product, we carried out the following experiments.

The DNA plasmid pMT2SM contains the adenovirus 5 major late promoter (MLP) and the SV40 ori enabling high levels of expression of foreign genes in transformed mammalian cells, such as SV-40-transformed Cos cells. Furthermore, the pMT2SM vector contains a Myc-tag (amino acids: EQKLISEEDL) which is in-frame with the foreign gene product. This Myc-tag enables the recognition of e.g. Apoptin-associating proteins by means of the Myc-tag-specific 9E10 antibody.

The pMT2SM vector expressing Myc-tagged AAP-4 cDNA was constructed as follows. The pACT-AAP-4 cDNA clone was digested with the restriction enzyme XhoI and the cDNA insert was isolated. The expression vector pMT2SM was digested with XhoI and treated with calf intestine alkline phosphatase and ligated to the isolated AAP-4 cDNA inserts. By sequence analysis, the pMT2SM constructs containing the AAP-4 cDNA in the correct orientation were identified.

The synthesis of Myc-tagged AAP-4 protein was analyzed by transfection of Cos cells with plasmid pMT2SM-AAP-4. As negative control, Cos cells were mock-transfected. Two days after transfection, the cells were lysed and Western-blot analysis was carried out using the Myc-tag-specific antibody 9E10.

The Cos cells transfected with pMT2SM-AAP-4 were proven to synthesise a specific Myc-tagged AAP-4 product with the size of approximately 60-65 kDa. As expected, the lysates of the mock-transfected Cos cells did not contain a protein product reacting with the Myc-tag-specific antibodies.

These results indicate that we have been able to isolate a cDNA that is able to produce a protein product with the ability to associate to the apoptosis-inducing protein Apoptin.

### Co-immunoprecipitation of Myc-tagged AAP-4 protein with Apoptin in a transformed mammalian cell system.

Next, we have analysed the association of Apoptin and the AAP-4 protein by means of co-immunoprecipitations using the Myc-tag-specific antibody 9E10. The 9E10 antibodies were shown not to bind directly to Apoptin, which enables the use of 9E10 for carrying out co-immunoprecipitations with (myc-tagged) Apoptin-associating proteins and Apoptin.

To that end, Cos cells were co-transfected with plasmid pCMV-VP3 encoding Apoptin and with plasmid pMT2SM-AAP-4. As a negative control, cells were transfected with pCMV-VP3 expressing Apoptin and a plasmid pcDNA3.1.LacZ-myc/His-LacZ encoding the myc-tagged beta-galactosidase, which does not associate with Apoptin.

Two days after transfection, the cells were lysed in a buffer consisting of 50 mM Tris (7.5), 250 mM NaCl, 5 mM EDTA, 0.1 % Triton X100, 1 mg/ml Na₄P₂O₇ and freshly added protease inhibitors such as PMSF, Trypsine-inhibitor, Leupeptine and Na3VO4. The specific proteins were immuno-precipitated as described by Noteborn et al. (1998) using the Myc-tag-specific antibodies 9E10, and analyzed by Western blotting.

Staining of the Western blot with 9E10 antibodies and 111.3 antibodies, which are specifically directed against myc-tag and Apoptin, respectively, showed that the "total" cell lysates contained the 16-kDa Apoptin product and the Myc-tagged AAP-4 protein or beta-galactosidase product. Immunoprecipitation of the Myc-tagged AAP-4 products was accompanied by the immuno-precipatation of Apoptin product of 16 kDa. In contrast, immunoprecipitation of myc-tagged beta-galactosidase did not result in a significant co-precipitation of the Apoptin protein. In addition, immunoprecipitation of the Apoptin protein, by means of a polyclonal antibody directed against the C-terminal part of Apoptin (Noteborn and Danen, unpublished results), was accompanied by the immunoprecipitation of the myc-tagged AAP-4 product of 60-65 kDa.

In total, three independent immunoprecipitation experiments were carried out, which all showed the associating ability of Apoptin to the AAP-4 protein.

These results indicate that the novel determined AAP-4 protein is able to specifically associate with Apoptin not only in the yeast background, but also in a mammalian transformed cellular system.

### Over-expression of the novel AAP-4 protein in human osteosarcoma Saos-2 cells, lacking functional p53, induces the apoptotic process.

We have examined whether AAP-4 carries apoptotic activity in Saos-2 cells. First, we have analysed the cellular localisation of the novel AAP-4 protein in human transformed cells. To that end, the human osteosarcoma-derived Saos-2 cells were transfected, as described by Danen-van Oorschot (1997), with plasmid pMT2SM-AAP-4 encoding the myc-tagged AAP-4 protein, respectively.

By indirect immunofluorescence using the myc-tag-specific antibody 9E10 and DAPI, which stains the nuclear DNA, it was shown that AAP-4 protein was present in the nucleus of the cell. Most often it co-localizes with the chromatin/DNA structures.

During tumor development, most of the tumors will lack functional tumor suppressor p53. Tumor cells lacking functional p53 are in general poor responders to (chemo)therapeutic agents. Therefore, it is of importance to prove whether AAP-4 can induce apoptosis in human tumor cells in the absence of functional p53.

To this end we examined whether (over)-expression of AAP-4 protein results in induction of apoptosis. Four days after transfection, the majority of AAP-4-positive cells were aberrantly stained with DAPI, which is indicative for induction of apoptosis (Telford, 1992, Danen-van Oorschot, 1997). Cells expressing Apoptin also underwent apoptosis, albeit slightly less than the AAP-4-producing cells, whereas as expected the cells synthesizing the non-apoptotic beta-galactosidase (LacZ) protein did not. The results are shown in Figure 3.

Co-expression of Apoptin and AAP-4 protein in human tumor cells, such as Saos-2 cells, results in a faster apoptotic process as expression of Apoptin or AAP-4 protein alone (Figure 3).

The fact that AAP-4 protein can induce apoptosis in p53-minus Saos-2 cells indicates that AAP-4 can induce p53-independent apoptosis. These results imply that AAP-4 can be used as anti-tumor agent in cases where other (chemo)therapeutic agents will fail. Furthermore, the finding that both Apoptin and AAP-4 induce a p53-independent pathway indicates that AAP-4 fits in the Apoptin-induced apoptotic pathway.

In conclusion, we have identified an Apoptin-associating protein, namely the novel AAP-4 protein, which is present in the nucleus and able to induce (p53-independent) apoptosis in human tumor cells.

### Over-expression of the novel AAP-4 protein in human osteosarcoma U20S cells, expressing wild-type p53, induces the apoptotic process.

We were also interested in examining the activity of AAP-4 in an additional cell line. To this end we have transfected human osteosarcoma-derived U20S cells expressing wild-type p53 with plasmid pMT2SM-AAP-4 encoding the myc-tagged AAP-4 protein. As control, U20S cells were transfected with the plasmid pCMV-LacZ encoding myc-tagged LacZ. Four or five days after transfection, the cells were fixed and analysed for myc-tagged AAP-4 expression by means of immunofluorescence using the myc-tag specific antibody 9E10 and for apoptotic activity by DAPI staining. U20S cells were stained aberrantly with DAPI whereas the cells containing myc-tagged LacZ were not, which is indicative for induction of AAP-4-specific apoptosis.

These experiments were repeated three times and clearly showed that AAP-4 alone also kills U20S tumor cells (figure 3). Thus absence or presence of p53 is irrelevant for AAP-4 activity (like Apoptin), which broadens the tumor-target spectrum of AAP-4.

### AAP-4 localizes in apoptotic structures.

One of the striking features of CAV-induced apoptosis is the "circular" Apoptin-positive structures occurring during the apoptotic process (Noteborn et al., 1994). These structures are visible in the nucleus at an early time point during apoptosis and become at a later stage more and more pronouncedly structured as circles or circular appearances or structures. We have now found that AAP-4 is located in the same "circular" structures as described for Apoptin. We have analyzed cells coexpressing both (myc-tagged) AAP-4 and Apoptin. Saos-2 cells were co-transfected with pMT2SN-AAP-4 and pCMV-Apoptin (Danen-van Oorschot, 1997). Indirect immunofluorescence, using both antibodies detecting AAP-4 or Apoptin and FITC- or rhodamine-labeled secondary antibody conjugates was carried out as described by Danen-van Oorschot, 1997a, proved that the "circular" structures contain both Apoptin and AAP-4 protein. Within these structures, Apoptin and AAP-4 partially colocalize with each other. It is the first cellular (derived from human (tumor) cells) protein that is associated with CAV- and/or Apoptin-associated apoptotic structures. A schematic representation of these structures is given in figure 4.

When AAP-4 was expressed alone in Saos-2 tumor cells, the staining pattern was diffusely punctuate nuclear staining in addition to large circular structures that lacked staining, giving the appearance of black holes in the nucleus. In contrast, normal VH10 cells expressing the AAP-4 construct showed only the diffuse nuclear staining, with no evidence for such black holes. These results strongly suggest a tumor-specific distribution of AAP-4 in the absence of Apoptin.

In conclusion, we have provided evidence that interference of specific factors with the function of AAP-4 proteins results in induction of apoptosis. Therapies based on induction of (p53-independent) apoptosis are possible utilising the interference with the function of AAP-4 proteins. An example of such an interfering factor is Apoptin. Another CAV-derived protein, which is known to induce apoptosis and also known to enhance Apoptin activity is VP2 (Noteborn et al., 1997).

### Utility of AAP-4 antisera

The best AAP-4 antibody of the two generated was selected for further use. We tested the efficacy of this antibody by transfecting primate COS-7 and human U20S cells with the AAP-4-myc construct. Western analysis showed that the approximately 60-65 kDa AAP-4-myc protein was detected strongly only in samples where the DNA was transfected. Similarly, in immunoprecipitation experiments, AAP-4-myc was also strongly detected. Finally, we could detect the presence of AAP-4 in the nucleus using this AAP-4 antibody in immunofluorescence analysis.

### Northern blot analysis

To examine whether AAP-4 was differentially expressed in tumor versus normal tissue, we tested a commercially-available Northern blot (Invitrogen, cat. No. #D310001) that contained tumor and normal tissue derived from the same patient, from a variety of tissue types.

There was a very large RNA, approximately 6 to 7 kb, expressed in normal brain tissue that was not present in the same amount of RNA from uninvolved brain of the same patient. However, in the normal brain sample we saw much smaller faint bands that might indicate the presence of splice variants.

### Cloning and sequence analysis of full-length AAP-4

A further sequence analysis of the human AAP-4 DNA sequence yielded the 5690 bp long nucleic acid sequence given in figure 5. An open reading frame was found in this nucleic acid sequence at position 236 to 2866. The deduced amino acid sequence is given in figure 6.

A protein domain called SET-domain was found in the amino acid sequence of the human AAP-4 protein. It spans the region of the amino acid 185 to amino acid 304.

The SET domain is a 130-amino acid, evolutionarily conserved sequence motif present in chromosomal proteins that function in modulating gene activities from yeast to mammals. Initially identified as members of the Polycomb- and trithorax-group (Pc-G and trx-G) gene families, these genes regulate the expression of the homeotic genes through a mechanism thought to involve some aspect of chromatin structure. Other proteins which have this motif also have additional domains or characteristics that support that suggestion that the SET domain is involved in chromatin-mediated gene regulation, and possibly in determining chromosome architecture. These observations implicate SET domain proteins as multifunctional chromatin regulators with activities in both eu- and heterochromatin (T. Jenuwein et al. 1998,Cell. Mol. Life Sci. 54, 80-93).

Recently, it has been demonstrated that SET domains are protein-protein interaction domains important for the activity of multicomponent complexes involved in transcriptional activation or repression or phosphorylation (T. Rozovskaia et al 2000, Oncogene 19, 351-357). SET domains are found in a number of proteins closely associated with human tumorigenesis such as HRX/ALL1/MLL/Htrx, MOZ and MMSET all of which are part of aberrant fusion proteins derived from chromosomal translocations found in a high percentage of human leukemias (S.Jacobson and L. Pillus 1999, Curr. Opinion in Gen & Dev. 9, 175-184).

It was not until the present invention that such a SET domain was identified in an Apoptin associating protein and therefore, affecting the functional activity of the SET domain of AAP-4 should have therapeutic effects against tumors. The SET domain can be used to identify substances which bind to the SET domain. This would be done by methods known to persons skilled in the art, e.g. by binding studies, where an AAP-4 peptide comprising the SET domain is bound to a matrix and it is tested whether test substances bind to the AAP-4 peptide, or by co-immunoprecipitation of an AAP-4 peptide comprising the SET domain with test substances using antibodies generated against the AAP-4 peptide comprising the SET domain. Test substances are for example small organic compounds derived e.g. from a compound library or peptides or proteins derived e.g. from a peptide library or from a natural source like a cell extract. The test substances are for example labelled for easier detection. The substances found to bind to the SET domain can either enhance or inhibit one or more effects of AAP-4. This is tested by measuring the apoptotic activity of AAP-4 as described above in the presence of said substances and by determining the nuclear localization of AAP-4 as described above in the presence of said substances.

### Description of the figures

Figure 1 shows the partial sequence of vector pMT2SM-AAP-4. The DNA sequence of the AAP-4 cDNA starts at position 12 of the DNA sequence and is indicated as "start AAP4 cDNA."

Figure 2 shows the amino-acid sequence of the analysed region of the Apoptin-associating clone AAP-4 (bold). In addition, the three C-terminal amino acids H-E-G of the multiple cloning site of pACT are given to illustrate that the AAP-4 amino acid sequence is in frame with the GAL4-activation domain. This feature proves that the AAP-4 region is indeed synthesised in yeast cells.

Figure 3 shows the apoptotic activity of AAP-4 protein and/or Apoptin in human osteosarcoma-derived Saos-2 cells and in human osteosarcoma U20S cells. (-): no apoptotic activity; (++): strong apoptotic activity; (+++): very strong apoptotic activity. In total three independent experiments have been carried out for both cell types.

Figure 4 shows a schematic representation of the nuclear "circular" apoptotic structures containing AAP-4 protein, which are visible in human tumor cells undergoing apoptosis.

Figure 5 shows the nucleic acid sequence of full-length AAP-4.

Figure 6 shows the amino acid sequence deduced from the nucleic acid sequence of figure 5.

Figure 7 shows the SET domain of the AAP-4 protein.

### REFERENCES

1. Bellamy, C.O.C., Malcomson, R.D.G., Harrison, D.J., and Wyllie, H. 1995. Cell death and disease: The biology and regulation of apoptosis. Seminars in Cancer Biology 6, 3-12.
2. Danen-Van Oorschot, A.A.A.M., Fischer, D.F., Grimbergen, J.M., Klein, B., Zhuang, S.-M., Falkenburg, J.H.F., Backendorf, C., Quax, P.H.A., Van der Eb, J.A., and Noteborn, M.H.M. (1997). Apoptin induces apoptosis in human transformed and malignant cells but not in normal cells. Proceedings National Academy Sciences, USA: 94, 5843-5847.
3. Danen-Van Oorschot, A.A.A.M, Den Hollander, A., Takayama, S., Reed, J., Van der Eb, A.J. and Noteborn, M.H.M. (1997a). BAG-1 inhibits p53-induced but not Apoptin-induced apoptosis. Apoptosis 2, 395-402.
4. Duke, R.C., Ocjius, D.M., Young, J, D-E. (1996). Cell suicide in health and disease. Scientific American December 1996, 48-55.
5. Durfee, T., Becherer, K., Chen, P.-L., Yeh,S.-H., Yang, Y., Kilburn, A.E., Lee, W.-H., and Elledge, S.J. (1993). The retinoblastoma protein associates with the protein phosphate type I catalytic subunit. Genes and Development 7, 555-569.
6. Earnshaw, W.C., 1995. Nuclear changes in apoptosis. Current Opinion in Cell Biology 7, 337-343.
7. Fields, S. and Song, O.K. (1989). A novel genetic system to detect protein-protein interactions. Nature 340, 245-246.
8. Hockenberry, D.M. (1994). Bcl-2 in cancer, development and apoptosis. Journal of Cell Science, Supplement 18, 51-55.
9. Hoffman, C.S. and Winston, F. (1987). A ten-minute DNA preparation from yeast efficiently releases autonomous plasmids for transformation of Escherichia coili. Gene 57, 267-272.
10. Kerr, J.F.R., Winterford, C.M., and Harmon, B.V. (1994). Apoptosis: Its significance in cancer and cancer therapy. Cancer 73, 2013-2026.
11. Klebe, R.J., Harriss, J.V., Sharp, Z.D., and Douglas, M.G. (1983). A general method for polyethylene-glycol-induced genetic transformation of bacteria and yeast. Gene 25, 333-341.
12. Levine, A.J. (1997). p53, the cellular gatekeeper for growth and division. Cell 88, 323-331.
13. Maniatis, T., Fritsch, E.F., and Sambrook, J. (1982). Molecular Cloning: A Laboratory Manual. CSHL Press, New York, USA.
14. McDonell T.J., Meyn, R.E., Robertson, L.E. (1995). Implications of apoptotic cell death regulation in cancer therapy. Seminars in Cancer Biology 6, 53-60.
15. Noteborn, M.H.M. (1996). PCT application WO 96/41191. Apoptin induces apoptosis in human transformed and malignant cells but not in normal cells as essential characteristic for the development of an anti-tumor therapy.
16. Noteborn, M.H.M., and De Boer, G.F. (1996). Patent USA/no. 030, 335.
17. Noteborn, M.H.M., De Boer, G.F., Van Roozelaar, D., Karreman, C., Kranenburg, O., Vos, J., Jeurissen, S., Zantema, A., Hoeben, R., Koch, G., Van Ormondt, H., and Van der Eb, A.J. (1991). Characterization of cloned chicken anemia virus DNA that contains all elements for the infectious replication cycle. Journal of Virology 65, 3131-3139.
18. Noteborn, M.H.M., and Pietersen, A. (1998). A gene delivery vehicle expressing the apoptosis-inducing proteins VP2 and/or Apoptin. PCT Application no. PCT/NL98/00213
19. Noteborn, M.H.M., Todd, D., Verschueren, C.A.J., De Gauw, H.W.F.M., Curran, W.L., Veldkamp, S., Douglas, A.J., McNulty, M.S., Van der Eb, A.J., and Koch, G. (1994). A single chicken anemia virus protein induces apoptosis. Journal of Virology 68, 346-351.
20. Noteborn, M.H.M., Verschueren, C.A.J., Koch, G., and Van der Eb, A.J. (1998). Simultaneous expression of recombinant baculovirus-encoded chicken anemia virus (CAV) proteins VP1 and VP2 is required for formation of the CAV-specific neutralizing epitope. Journal General Virology, 79, 3073-3077.
21. Noteborn, M.H.M., and Zhang, Y. (1998). Methods and means for determining the transforming capability of agents, for determining the predisposition of cells to become transformed and prophylactic treatment of cancer using Apoptin-like activity. PCT Application no. PCT/NL98/00457
22. Noteborn, M.H.M., Danen-van Oorschot, A.A.A.M., Van der Eb, A.J. (1998a). Chicken anemia virus: Induction of apoptosis by a single protein of a single-stranded DNA virus. Seminars in Virology 8, 497-504.
23. Paulovich, A.G., Toczyski, D., Hartwell, H. (1997). When checkpoints fail. Cell 88, 315-321.
24 Pietersen, A.M., Van der Eb, M.M., Rademaker, H.J., Van den Wollenberg, D.J.M., Rabelink, M.J.W.E., Kuppen, P.J.K., Van Dierendonck, J.H., Van Ormondt, H., Masman, D., Van de Velde, C.J.H., Van der Eb, Hoeben, R.C., and Noteborn, M.H.M. (1999). Specific tumor-cell killing with adenovirus vectors containing the Apoptin gene. Gene Therapy 6, 882-892.
25. Rose, M.D., Winston, F., and Hieter, P. (1990). Methods in yeast genetics. A laboratory course manual. Cold Spring Harbor Laboratory Press, Cold Spring Harbor, New York, USA.
26. Sachs, L. and Lotem, J. (1993). Control of programmed cell death in normal and leukemia cells: New implications for therapy. Blood 82, 15-21.
27. Sanger, F., Nicklen, S., and Coulsen, A.R. (1977). DNA sequencing with chain-terminating inhibitors. Proceedings National Academic Sciences USA 74, 5463-5467.
28. Steller, H. (1995). Mechanisms and genes of cellular suicide. Science 267, 1445-1449.
29. Telford, W.G., King, L.E., Fraker, P.J. (1992). Comparative evaluation of several DNA binding dyes in the detection of apoptosis-associated chromatin degradation by flow cytometry. Cytometry 13, 137-143.
30. Teodoro, J.G. and Branton, P.E. (1997). Regulation of apoptosis by viral gene products. Journal of Virology 71, 1739-1746.
31. Thompson, C.B. (1995). Apoptosis in the pathogenesis and treatment of disease. Science 267, 1456-1462.
32. White, E. (1996). Life, death, and the pursuit of apoptosis. Genes and development 10, 1-15.
33. Wyllie, A.H. (1995). The genetic regulation of apoptosis. Current Opinion in Genetics and Development 5, 97-104.
34. Wyllie, A.H., Kerr, J.F.R., Currie, A.R. (1980). Cell death: The significance of apoptosis. International Review of Cytology 68, 251-306.
35. Yang, X., Hubbard, E.J.A., and Carlson, M. (1992). A protein kinase substrate identified by the two-hybrid system. Science 257, 680-682.
36. Zhuang, S.-M., Landegent, J.E., Verschueren, C.A.J., Falkenburg, J.H.F., Van Ormondt, H., Van der Eb, A.J., Noteborn, M.H.M. (1995). Apoptin, a protein encoded by chicken anemia virus, induces cell death in various human hematologic malignant cells *in vitro.* Leukemia 9 S1, 118-120.
37. Zhuang, S.-M., Shvarts, A., Van Ormondt, H., Jochemsen, A.-G., Van der Eb, A.J., Noteborn, M.H.M. (1995). Apoptin, a protein derived from chicken anemia virus, induces a p53-independent apoptosis in human osteosarcoma cells. Cancer Research 55, 486-489.
38. Evans GI, Lewis GK, Ramsay G, Bishop JM, (1985), Isolation of monoclonal antibodies specific for human c-myc proto-oncogene product. Mol. Cell. Biol. Dec 5 (12), 3610-3616
39. T. Jenuwein et al. (1998),Cell. Mol. Life Sci. 54, 80-93
40. T. Rozovskaia et al (2000),Oncogene 19, 351-357
41. S.Jacobson and L. Pillus (1999), Curr. Opinion in Gen & Dev. 9, 175-184

## Claims

1. An isolated or recombinant nucleic acid or functional equivalent or functional fragment thereof encoding an Apoptin-associating proteinaceous substance capable of providing nuclear localisation and apoptosis.

2. A nucleic acid according to claim 1 derived from a cDNA library.

3. A nucleic acid according to claim 1 or 2 wherein said cDNA library comprises human cDNA.

4. A nucleic acid according to anyone of claims 1 to 3 capable of hybridising to a nucleic acid molecule encoding an Apoptin-associating proteinaceous substance as shown in figure 1 or 5.

5. A nucleic acid according to anyone of claims 1 to 4 being at least 60% homologous to a nucleic acid molecule, or to a functional equivalent or functional fragment thereof, encoding an Apoptin-associating proteinaceous substance as shown in figure 1 or 5.

6. A vector comprising a nucleic acid according to anyone of claims 1 to 5.

7. A vector according to claim 6 comprising a gene-delivery vehicle.

8. A host cell comprising a nucleic acid according to anyone of claims 1 to 5 or a vector according to claim 6 or 7.

9. A host cell according to claim 8 which is a eukaryotic cell such as a yeast cell or a vertebrate cell.

10. An isolated or recombinant Apoptin-associating proteinaceous substance capable of providing nuclear localisation and apoptosis.

11. A proteinaceous substance according to claim 10 encoded by a nucleic acid according to anyone of claims 1 to 5.

12. A proteinaceous substance according to claim 10 or 11 comprising at least a part of an amino acid sequence as shown in figure 2 or 6 or a functional equivalent or functional fragment thereof.

13. An isolated or synthetic antibody specifically recognising a proteinaceous substance or functional equivalent or functional fragment thereof according to anyone of claims 10 to 12.

14. A proteinaceous substance specifically recognisable by an antibody according to claim 13.

15. Use of a nucleic acid according to anyone of claims 1 to 5, a vector according to claims 6 or 7, a host cell according to claim 8 or 9, a proteinaceous substance according to anyone of claims 10 to 12 or 14 for the induction of apoptosis.

16. Use according to claim 15 wherein said apoptosis is p53-independent.

17. Use according to claim 15 or 16 further comprising use of a nucleic acid encoding Apoptin or a functional equivalent or fragment thereof or use of Apoptin or a functional equivalent or fragment thereof.

18. A pharmaceutical composition comprising a nucleic acid according to anyone of claims 1 to 5, a vector according to claims 6 or 7, a host cell according to claim 8 or 9, a proteinaceous substance according to anyone of claims 10 to 12 or 14.

19. A pharmaceutical composition according to claim 18 further comprising a nucleic acid encoding Apoptin or a functional equivalent or fragment thereof or Apoptin or a functional equivalent or fragment thereof.

20. A pharmaceutical composition according to claim 18 or 19 for the induction of apoptosis.

21. A pharmaceutical composition according to claim 20 wherein said apoptosis is p53-independent.

22. A pharmaceutical composition according to anyone of claims 18 to 21 for the treatment of a disease where enhanced cell proliferation or decreased cell death is observed.

23. A pharmaceutical composition according to claim 22 wherein said disease comprises cancer or auto-immune disease.

24. A method for treating an individual carrying a disease where enhanced cell proliferation or decreased cell death is observed comprising treating said individual with a pharmaceutical composition according to anyone of claims 18 to 23.

25. An isolated or recombinant nucleic acid encoding a proteinaceous substance comprising the amino acid sequence as shown in figure 7

26. An assay to identify a putative effector of the activity of the proteinaceous substance encoded by a nucleic acid as shown in figure 5 comprising bringing in contact a proteinaceous substance comprising amino acid 185-304 of the amino acid sequence shown in figure 6 with said effector and determining the binding of said effector.
